# EUROPEAN PATENT SPECIFICATION

(11) **EP 3 730 944 B1**
(45) Date of publication and mention of the grant of the patent: **29.12.2021**
(21) Application number: 20177892.5
(22) Date of filing: 13.04.2017
(51) Int. Cl.: G01N 33/68, A61P 9/00, A61P 9/12, A61P 15/00, G16H 10/40, G16H 15/00, G16H 20/10

(54) **CARDIAC TROPONIN I DETECTION DURING PREGNANCY FOR CARDIOVASCULAR DISEASE IDENTIFICATION AND RISK ASSESSMENT**
DETEKTION VON KARDIALEM TROPONIN I WÄHREND DER SCHWANGERSCHAFT ZUR IDENTIFIZIERUNG VON HERZKREISLAUFERKRANKUNGEN UND ZUR RISIKOBEWERTUNG
DÉTECTION DE TROPONINE I CARDIAQUE PENDANT LA GROSSESSE POUR L'IDENTIFICATION DE MALADIES CARDIOVASCULAIRES ET D'ÉVALUATION DE RISQUES

(30) Priority: 13.04.2016 US 201662322111 P
(43) Date of publication of application: 28.10.2020
(62) Divisional of application: 17735252.3
(73) Proprietor: Abbott Laboratories, Abbott Park, IL 60064 (US)
(72) Inventor: BESHIRI, Agim, Abbott Park, IL 60064 (US); LIM, Yee Cherng, Abbott Park, IL 60064 (US); NOOR, Eliza Mohd, Abbott Park, IL 60064 (US); SONG QUEK, Yek, Abbott Park, IL 60064 (US); RAVICHANDRAN, Jeganathan, Abbott Park, IL 60064 (US); SICKAN, Jaganathan, Abbott Park, IL 60064 (US); SURESH, Kumar, Abbott Park, IL 60064 (US); VIGNESWARAN, Ramakrishnan, Abbott Park, IL 60064 (US); YUAN WOON, Shu, Abbott Park, IL 60064 (US)
(74) Representative: Modiano, Gabriella Diana

(56) References cited:
- WO-A1-2011/157445
- WO-A2-2007/114947
- CEMAL ATALAY ET AL: "The effect of magnesium sulfate treatment on serum cardiac troponin I levels in preeclamptic women", ACTA OBSTETRICIA AND GYNECOLOGICA SCANDINAVICA., vol. 84, no. 7, 1 July 2005 (2005-07-01), pages 617-621, XP055395369, DK ISSN: 0001-6349, DOI: 10.1111/j.0001-6349.2005.00667.x
- FLEMING S M ET AL: "Cardiac troponin I in pre-eclampsia and gestational hypertension", BJOG: AN INTERNATIONAL JOURNAL OF OBSTETRICS AND GYNAECOLOGY, WILEY-BLACKWELL PUBLISHING LTD, GB, vol. 107, no. 11, 1 November 2000 (2000-11-01), pages 1417-1420, XP002658333, ISSN: 1470-0328
- PERGIALIOTIS VASILIOS ET AL: "Maternal cardiac troponin levels in pre-eclampsia: a systematic review", THE JOURNAL OF MATERNAL FETAL & NEONATAL MEDICINE : THE OFFICIAL JOURNAL OF THE EUROPEAN ASSOCIATION OF PERINATAL MEDICINE, THE FEDERATION OF ASIA AND OCEANIA PERINATAL SOCIETIES, THE INTERNATIONAL SOCIETY OF PERINATAL OBSTETRICIANS, INFORMA HEALTHCA, vol. 29, no. 20, 8 January 2016 (2016-01-08), pages 3386-3390, XP009195121, ISSN: 1057-0802
- F. S. APPLE ET AL: "Determination of 19 Cardiac Troponin I and T Assay 99th Percentile Values from a Common Presumably Healthy Population", CLINICAL CHEMISTRY., vol. 58, no. 11, 14 September 2012 (2012-09-14), pages 1574-1581, XP055395372, WASHINGTON, DC. ISSN: 0009-9147, DOI: 10.1373/clinchem.2012.192716
- SHIVVERS ET AL: "Maternal cardiac troponin I levels during normal labor and delivery", AMERICAN JOURNAL OF OBSTETRICS & GYNECO, MOSBY, ST LOUIS, MO, US, vol. 180, no. 1, 1 January 1999 (1999-01-01), pages 122-127, XP005142466, ISSN: 0002-9378, DOI: 10.1016/S0002-9378(99)70161-4

## Description

### FIELD OF THE INVENTION

The invention provides systems for determining the cardiac troponin I concentration in a sample from a pregnant subject (e.g., woman in the first, second, or third trimester) and determining whether the pregnant subject has, or is at risk of, developing cardiovascular disease, such as gestational hypertension.

### BACKGROUND

Pregnancy induced hypertension (PIH) disorders include preeclampsia (PE) and gestational hypertension (GH). Preeclampsia is characterized as the combination of high blood pressure (hypertension), swelling (edema), and protein in the urine (albuminuria, proteinuria) developing after the 20th week of pregnancy. Preeclampsia ranges in severity from mild to severe. The mild form is often called proteinuric pregnancy-induced hypertension or proteinuric gestational hypertension. Gestational hypertension is generally characterized as the acute onset of hypertension in pregnancy or the early puerperium without proteinuria or abnormal edema and resolving within 10 days after delivery.

Individuals at increased risk of developing preeclampsia and eclampsia include primigravidas and women with multiple gestations, molar pregnancy or fetal hydrops, chronic hypertension or diabetes, or a personal or family history of eclampsia or preeclampsia. Preeclampsia and gestational hypertension are forms of PIH. The standard therapy for PIH is fetus delivery, often at the expense of fetal well-being. Prophylactic strategies to prevent PIH, including calcium supplementation and aspirin therapy, have been mostly unsuccessful. One reason these therapies have failed is that the absence of screening tests limits the ability to administer the therapeutic interventions early enough to modify pregnancy outcomes. For example, diagnosing PE by the appearance of edema and proteinuria alone is unreliable as edema is common in normal pregnancies and measurable proteinuria usually occurs only after hypertension is manifested. Therefore, such a test lacks specificity and fails to detect GDM or PIH prior to manifestation of the disease in the third trimester of pregnancy. Thus, there exists a need for diagnostic methods and systems that lead to early implementation of therapy and improved pregnancy outcomes for women at risk for PIH.

WO 2011/157445 relates to the prognosis and risk assessment in pregnant women to develop pregnancy-induced hypertension and/or preeclampsia by the determination of marker levels. It was known that cTnl levels are increased during preeclampsia, eclampsia and gestational hypertension (see e.g. Cemal Atalay et Al.. Acta Obstetricia and Gynecologica Scandinavica. vol. 84. no. 7, July 2005, p.617-621; and Pcrgialiotis Vasilios et Al., The Journal of Maternal Fetal & Neonatal Medicine, vol. 29, no. 20. January 2016, p.3386-3390).

### SUMMARY OF THE INVENTION

The present disclosure provides systems for determining the cardiac troponin I concentration in a sample from a pregnant subject (e.g., woman in the first, second, or third trimester) and determining that, or generating a report that, the sample concentration is, or is not, higher than a 99^{th} percentile cTnI concentration from a corresponding first, second, or third trimester uncomplicated pregnancy control group, thereby determining if the pregnant subject has or is at risk of developing cardiovascular disease, such as gestational hypertension.

In certain embodiments, provided herein are systems comprising: a) a report that: i) provides a sample concentration of cTnI from a first, second, or third trimester for a pregnant woman as tested by a first assay; and ii) provides the 99^{th} percentile cTnI concentration for a first, a second, and/or a third trimester uncomplicated pregnancy control group (e.g., as tested by the first assay); wherein the report indicates that the pregnant woman has, or is at risk of developing cardiovascular disease (CVD) or a complication of CVD, if the sample concentration of cTnI is higher than the corresponding uncomplicated pregnancy control group; and b) a computer for generating the report, wherein the computer comprises: i) a computer processor for receiving, processing, and communicating data, ii) a storage component for storing data which contains a reference database containing the 99^{th} percentile cTnI concentration for the first, second, and third trimester uncomplicated pregnancy control group; and iii) a computer program, embedded within the computer processor, which is configured to process the sample concentration of cTnI in the context of the reference database to determine, as an outcome, if the pregnant woman has or is at risk for developing cardiovascular disease or a complication of cardiovascular disease.

In certain embodiments, the 99^{th} percentile cTnI concentration for the first trimester as measured by the first assay is, or about, 4.0 pg/ml (e.g., 3.8, 3.9, 4.0, 4.1, or 4.2 pg/ml), the 99^{th} percentile cTnI concentration for the second trimester as measured by the first assay is, or about, 4.0 pg/ml (e.g., 3.8, 3.9, 4.0, 4.1, or 4.2 pg/ml), and the 99^{th} percentile cTnI concentration for the third trimester as measured by the first assay is, or about, 6.0 pg/ml (e.g., 5.8, 5.9, 6.0, 6.1, or 6.2 pg/ml). In certain embodiments, the report provides the 99^{th} percentile cTnI concentration for all three of the first, the second, and the third trimester uncomplicated pregnancy control group as tested by the first assay. In additional embodiments, the systems further comprise: a cardiovascular disease (CVD) therapeutic.

In certain embodiments, the 99^{th} percentile cTnI concentration for the first trimester as measured by the first assay is, or is less than about 9.0 pg/ml (e.g., 9.0, 8.9, 8.8, 8.7, 8.6, 8.5, 8.4, 8.3, 8.2, 8.1, 8.0, 7.9, 7.8, 7.7, 7.6, 7.6, 7.5, 7.4, 7.3, 7.2, 7.1, 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml)), the 99^{th} percentile cTnI concentration for the second trimester as measured by the first assay is, or is less than about 9.0 pg/ml (e.g., 9.0, 8.9, 8.8, 8.7, 8.6, 8.5, 8.4, 8.3, 8.2, 8.1, 8.0, 7.9, 7.8, 7.7, 7.6, 7.6, 7.5, 7.4, 7.3, 7.2, 7.1, 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml), and the 99^{th} percentile cTnI concentration for the third trimester as measured by the first assay is, or is less than about, 9.0 pg/ml (e.g., 9.0, 8.9, 8.8, 8.7, 8.6, 8.5, 8.4, 8.3, 8.2, 8.1, 8.0, 7.9, 7.8, 7.7, 7.6, 7.6, 7.5, 7.4, 7.3, 7.2, 7.1, 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml). In certain embodiments, the report provides the 99^{th} percentile cTnI concentration for all three of the first, the second, and the third trimester uncomplicated pregnancy control group as tested by the first assay. In additional embodiments, the systems further comprise: a cardiovascular disease (CVD) therapeutic.

In certain embodiments, the 99^{th} percentile cTnI concentration for the first trimester as measured by the first assay is, or is less than about 8.0 pg/ml (e.g., 8.0, 7.9, 7.8, 7.7, 7.6, 7.6, 7.5, 7.4, 7.3, 7.2, 7.1, 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml)), the 99^{th} percentile cTnI concentration for the second trimester as measured by the first assay is, or is less than about 8.0 pg/ml (e.g., 8.0, 7.9, 7.8, 7.7, 7.6, 7.6, 7.5, 7.4, 7.3, 7.2, 7.1, 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml), and the 99^{th} percentile cTnI concentration for the third trimester as measured by the first assay is, or is less than about, 8.0 pg/ml (e.g., 8.0, 7.9, 7.8, 7.7, 7.6, 7.6, 7.5, 7.4, 7.3, 7.2, 7.1, 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml). In certain embodiments, the report provides the 99^{th} percentile cTnI concentration for all three of the first, the second, and the third trimester uncomplicated pregnancy control group as tested by the first assay. In additional embodiments, the systems further comprise: a cardiovascular disease (CVD) therapeutic.

In certain embodiments, the 99^{th} percentile cTnI concentration for the first trimester as measured by the first assay is, or is less than about 7.0 pg/ml (e.g., 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml)), the 99^{th} percentile cTnI concentration for the second trimester as measured by the first assay is, or is less than about 7.0 pg/ml (e.g., 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml), and the 99^{th} percentile cTnI concentration for the third trimester as measured by the first assay is, or is less than about, 7.0 pg/ml (e.g., 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml). In certain embodiments, the report provides the 99^{th} percentile cTnI concentration for all three of the first, the second, and the third trimester uncomplicated pregnancy control group as tested by the first assay. In additional embodiments, the systems further comprise: a cardiovascular disease (CVD) therapeutic.

In certain embodiments, the 99^{th} percentile cTnI concentration for the first trimester as measured by the first assay is, or is less than about 6.0 pg/ml (e.g., 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml)), the 99^{th} percentile cTnI concentration for the second trimester as measured by the first assay is, or is less than about 6.0 pg/ml (e.g., 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml), and the 99^{th} percentile cTnI concentration for the third trimester as measured by the first assay is, or is less than about, 6.0 pg/ml (e.g., 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml). In certain embodiments, the report provides the 99^{th} percentile cTnI concentration for all three of the first, the second, and the third trimester uncomplicated pregnancy control group as tested by the first assay. In additional embodiments, the systems further comprise: a cardiovascular disease (CVD) therapeutic.

In certain embodiments, the 99^{th} percentile cTnI concentration for the first trimester as measured by the first assay is, or is less than about 7.5 pg/ml (e.g., 7.5, 7.4, 7.3, 7.2, 7.1, 7.0, 6.9, 6.8, 6.7, 6.6, 6.5 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml)), the 99^{th} percentile cTnI concentration for the second trimester as measured by the first assay is, or is less than about 4.6 pg/ml (e.g., 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml), and the 99^{th} percentile cTnI concentration for the third trimester as measured by the first assay is, or is less than about, 6.0 pg/ml (e.g., 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 pg/ml). In certain embodiments, the report provides the 99^{th} percentile cTnI concentration for all three of the first, the second, and the third trimester uncomplicated pregnancy control group as tested by the first assay. In additional embodiments, the systems further comprise: a cardiovascular disease (CVD) therapeutic.

In some embodiments, provided herein are systems comprising: a) a report that: i) provides a sample concentration of cTnI from a first, second, or third trimester for a pregnant subject (e.g., woman) as tested by a first assay; and ii) provides the 99^{th} percentile cTnI concentration for a first, a second, and/or a third trimester uncomplicated pregnancy control group as tested by the first assay; wherein the report indicates that the pregnant woman has, or is at risk of developing cardiovascular disease (CVD) or a complication of CVD, if the sample concentration of cTnI is higher than the corresponding uncomplicated pregnancy control group; and b) a cardiovascular disease therapeutic.

In some embodiments, provide herein is a method of assessing whether a pregnant woman is at risk of developing cardiovascular disease (such as preeclampsia or eclampsia) during pregnancy, the method comprising the steps of:
a) assessing the level of cardiac troponin I (cTnI) in a sample obtained from a pregnant woman in a first, second or third trimester of pregnancy; and
a) comparing the level of cTnI in step a) with a level of cTnI determined from a sample obtained earlier in the pregnancy of the pregnant woman and determining if the pregnant woman is at risk for cardiovascular disease if the level of cTnI obtained from the earlier obtained sample has at least doubled from the level of cTnI determined in the subsequent sample obtained from step a). Specifically, it has been discovered that when the levels of cTnI obtained from a later obtained sample (which may be referred to herein as "one or more subsequent samples) have at least doubled from the levels of cTnI obtained from one or more samples obtained from a pregnant woman earlier (in time) in her pregnancy (such samples referred to herein as a "first" or "earlier" sample), then the pregnant woman as at least a 9-fold risk of developing cardiovascular disease (such as preclampsia or eclampsia) during her pregnancy. For example, if the levels of cTnI determined in a sample obtained during the second trimester of a pregnancy (a subsequent sample) have at least doubled from the levels of cTnI determined in a sample during the first trimester of pregnancy (a first or earlier sample), then the woman has an at least 9-fold risk of developing cardiovascular disease during her pregnancy. By way of another example, if the levels of cTnI determined in a sample obtained during the third trimester of a pregnancy (a subsequent sample) have at least doubled from the levels of cTnI determined in a sample obtained during the second trimester of pregnancy (a first or earlier sample), then the woman has an at least 9-fold risk of developing cardiovascular disease during her pregnancy. By way of yet another example, if the levels of cTnI determined in a sample during the third trimester of a pregnancy (a subsequent sample) have at least doubled from the levels of cTnI determined in a sample obtained during the first trimester of pregnancy (a first or earlier sample), then the woman has an at least 9-fold risk of developing cardiovascular disease during her pregnancy.
The (subsequent) sample obtained from a pregnant woman in step a) can be obtained at any time after the earlier or first sample is obtained. Specifically, the one or more subsequent samples can be obtained at any time after the first or earlier samples are obtained. For example, the one or more subsequent samples can be obtained 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks or 35 weeks after the first or earlier samples are obtained from the pregnant woman.
In some embodiments, a pregnant woman diagnosed as being at risk for a cardiovascular disease pursuant to the above method is prescribed one or more anti-hypertensive agents.

In some embodiments, provide herein is a method of assessing whether a pregnant woman is at risk of developing cardiovascular disease (such as preeclampsia or eclampsia) during pregnancy, the method comprising the steps of:
b) determining a level of cardiac troponin I (cTnI) in a first sample obtained from a pregnant woman in the first, second or third trimester of pregnancy;
c) determining a level of cTnI in one or more subsequent samples obtained from a pregnant woman in the first, second or third trimester of pregnancy, wherein the one or more subsequent samples are obtained later in time then the first sample; and
d) comparing the level of cTnI in the first sample and one or more subsequent samples and determining if the pregnant woman is at risk for cardiovascular disease if the level of cTnI from the one or more subsequent samples have at least doubled from the level of cTnI determined in the first sample. Specifically, it has been discovered that when the levels of cTnI obtained from one or more subsequent samples has at least doubled from the levels of cTnI obtained from a first sample then the pregnant woman as at least a 9-fold risk of developing cardiovascular disease (such as preclampsia or eclampsia) during her pregnancy. The one or more subsequent samples can be obtained at any time after the first sample is obtained. For example, the one or more subsequent samples can be obtained 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks or 35 weeks after the first sample is obtained from the pregnant woman.
In some embodiments, a pregnant woman diagnosed as being at risk for a cardiovascular disease pursuant to the above method is prescribed one or more anti-hypertensive agents.

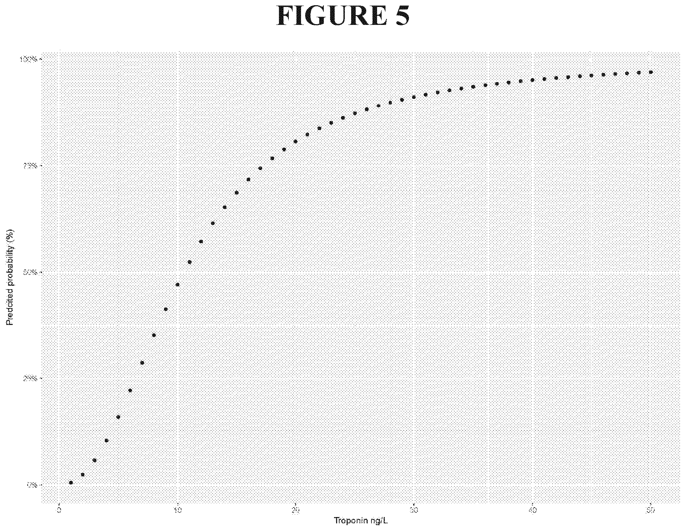

As shown in Figure 5, in some embodiments, provide herein is a method of assessing whether a pregnant woman is at risk of developing cardiovascular disease (such as preeclampsia or eclampsia) during pregnancy, the method comprising the steps of:
a) assessing the level of cardiac troponin I (cTnI) in one or more samples obtained from a pregnant woman at any time during one or more of her first, second or third trimesters of pregnancy; and
b) determining that the pregnant woman has at least a fifty percent (50%) risk of developing a cardiovascular disease during her pregnancy if the level of cTnI in the sample is at least 10 pg/mL.

In certain embodiments, the woman has a seventy-five percent (75%) risk of developing cardiovascular disease if the level of cTnI in the sample is at least 20 pg/mL. In certain embodiments, the woman has a ninety percent (90%) risk of developing cardiovascular disease if the level of cTnI in the sample is at least 30 pg/mL.

In some embodiments, a pregnant woman diagnosed as being at risk for a cardiovascular disease pursuant to the above system is prescribed one or more anti-hypertensive agents.

### BRIEF DESCRIPTION OF THE FIGURES

Figure 1A is a graph which shows the number of study samples in a normal uncomplicated pregnancy group as described in Example 1. Figure 1B is a graph which shows the total number of normal healthy subjects and complicated pregnancy subjects as described in Example 1.
Figure 2A is a graph which shows the mean value of Troponin I of an uncomplicated pregnancy group in different gestational categories (pg/mL) as described in Example 1. Figure 2B is a graph which shows the 99th percentile value of Troponin I for an uncomplicated pregnancy group in different gestational categories (pg/mL) as descried in Example 1.
Figure 3A is a graph which shows the mean value of troponin I compared with the normal group (pg/mL) as described in Example 1. Figure 3B is a graph which shows the 99th percentile value of troponin I compared with the normal group (pg/mL), as described in Example 1.

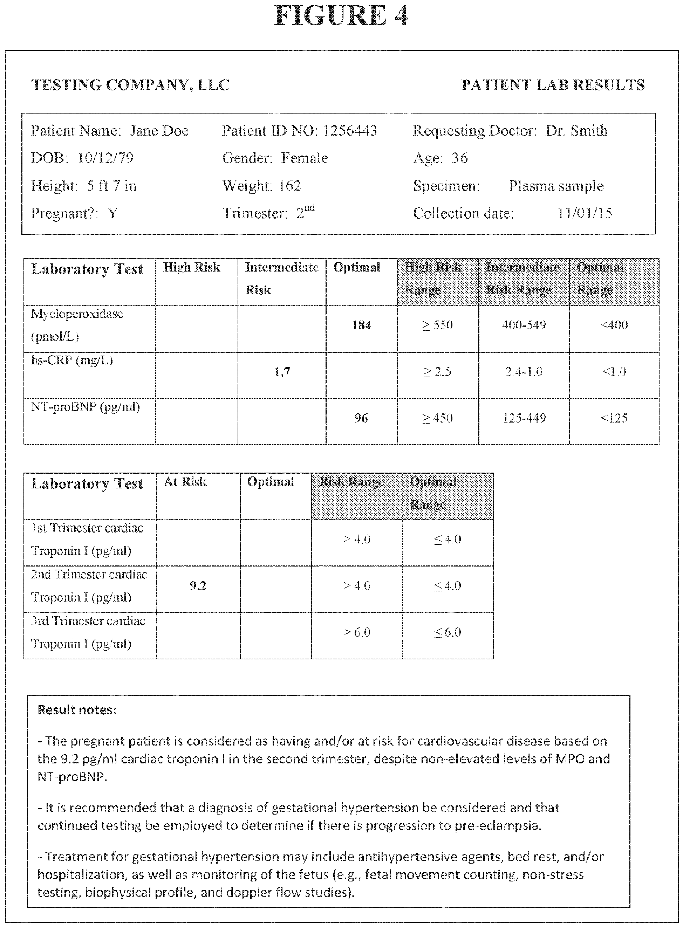

Figure 4 is an exemplary patient sample report.
Figure 5 is an expression of the predicted probability of developing PIH, where a doubling of hsTnI concentration has a 9 fold increase to the predicted probability of developing PIH.

### DEFINITIONS

As used herein, the terms "cardiovascular disease" (CVD) or "cardiovascular disorder" are terms used to classify numerous conditions affecting the heart, heart valves, and vasculature (e.g., veins and arteries) of the body and encompass diseases and conditions including, but not limited to arteriosclerosis, atherosclerosis, myocardial infarction, acute coronary syndrome, angina, congestive heart failure, aortic aneurysm, aortic dissection, iliac or femoral aneurysm, pulmonary embolism, primary hypertension, gestational hypertension, pre-eclampsia, atrial fibrillation, stroke, transient ischemic attack, systolic dysfunction, diastolic dysfunction, myocarditis, atrial tachycardia, ventricular fibrillation, endocarditis, arteriopathy, vasculitis, atherosclerotic plaque, vulnerable plaque, acute coronary syndrome, acute ischemic attack, sudden cardiac death, peripheral vascular disease, coronary artery disease (CAD), peripheral artery disease (PAD), and cerebrovascular disease.

The term "cardiovascular event," as used herein, refers to the manifestation of an adverse condition in a subject brought on by cardiovascular disease, such as sudden cardiac death or acute coronary syndromes including, but not limited to, myocardial infarction, gestational hypertension, pre-eclampsia, unstable angina, aneurysm, or stroke. The term "cardiovascular event" can be used interchangeably herein with the term "cardiovascular complication." While a cardiovascular event can be an acute condition, it can also represent the worsening of a previously detected condition to a point where it represents a significant threat to the health of the subject, such as the enlargement of a previously known aneurysm or the increase of hypertension to life threatening levels.

As used herein, the term "diagnosis" "diagnosis" can encompass determining the nature of disease in a subject, as well as determining the severity and probable outcome of disease or episode of disease and/or prospect of recovery (prognosis). "Diagnosis" can also encompass diagnosis in the context of rational therapy, in which the diagnosis guides therapy, including initial selection of therapy, modification of therapy (e.g., adjustment of dose and/or dosage regimen or lifestyle change recommendations), and the like.

The terms "individual," "host," "subject," and "patient" are used interchangeably herein, and generally refer to a pregnant mammal, including, but not limited to, pregnant primates, including simians and humans, equines (e.g., horses), canines (e.g., dogs), felines, various domesticated livestock (e.g., ungulates, such as swine, pigs, goats, sheep, and the like), as well as domesticated pets and animals maintained in zoos. In some embodiments, the subject is specifically a human female subject that is pregnant.

Before the present invention is further described, it is to be understood that this invention is not limited to particular embodiments described, as such may, of course, vary. It is also to be understood that the terminology used herein is for the purpose of describing particular embodiments only, and is not intended to be limiting.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a sample" includes a plurality of such samples and reference to a specific protein includes reference to one or more specific proteins and equivalents thereof known to those skilled in the art, and so forth.

### DETAILED DESCRIPTION OF THE INVENTION

The present disclosure provides systems and methods for determining the cardiac troponin I concentration in a sample from a pregnant woman (in the first, second, or third trimester) and determining that, or generating a report that, the sample concentration is, or is not, higher than a 99^{th} percentile cTnI concentration from a corresponding first, second, or third trimester uncomplicated pregnancy control group, thereby determining if the pregnant subject has or is at risk of developing cardiovascular disease, such as gestational hypertension.

Pregnancy stresses the myocardium and cardiovascular system increasing the circulating volume by 30 to 50 percent and the heart rate to ensure adequate fetal blood flow. Labor and delivery increases the cardiac workload further, especially during the third stage of labor, and compromises blood flow to the myocardium. Hypertensive disorders of pregnancy contribute significantly to maternal and peripartum mortality and morbidity. There is evidence to suggest that the hypertensive disorders of pregnancy, superimposed on the maternal cardiovascular adaptations required in normal pregnancy, have implications on the myocardial function (such as, e.g., left ventricular mechanics and the ultra-structure of the intra-myocardial vessels and cardiac myocytes). The adverse effects on the cardiovascular system in hypertensive disorders of pregnancy include, for example, hypertensive-induced left ventricular hypertrophy followed by both systolic and diastolic heart failure.

### I. Eclampsia, Preeclampsia, and Gestational Hypertension

In certain embodiments, the present disclosure provides methods and systems for diagnosing and reporting cardiovascular diseases during pregnancy, such as eclampsia, preeclampsia, and gestational hypertension during pregnancy. Preeclampsia is a rapidly progressive condition, characterized by the occurrence of high blood pressure and abnormal levels of protein in the urine (proteinuria). Eclampsia is a more severe form of preeclampsia that is also characterized by seizures. Gestational hypertension is hypertension in pregnancy without proteinuria, and it may be a less severe form of or a precursor to preeclampsia. Preeclampsia and gestational hypertension may be further classified as mild or severe depending upon the severity of the clinical symptoms. Such hypertension-related disorders are collectively referred to herein as "pregnancy-induced hypertension" or "PIH."

Typically, clinical symptoms of PIH occur in the late second trimester or in the third trimester of pregnancy, although symptoms may occur earlier in pregnancy. In certain embodiments, the methods and systems disclosed herein allow PIH to be diagnosed and reported earlier, such as in the first trimester (e.g., such that meaningful therapeutic intervention or earlier pregnancy termination may be accomplished). PIH may be superimposed over other forms of hypertension, such as essential and secondary hypertension, that exist prior to, or develop early in, pregnancy. An increased risk for PIH is associated with a variety of factors, including but not limited to, first time pregnancies, when there is a large interval between pregnancies, pregnant women under the age of 20 or over the age of 35, women of black race, multi-gestational pregnancies, women who have conceived through *in vitro* fertilization ("IVF"), women who have had a prior pregnancy with PIH, women who have had a prior pregnancy conceived with a different partner, women with a family history of PIH or high blood pressure or diabetes, women who are of higher than normal weight or body mass index prior to pregnancy, women with under-nutrition, women with a personal history of polycystic ovarian syndrome, insulin resistance or diabetes, hypertension, renal (kidney) disease, rheumatoid arthritis, systemic lupus erythematosus or other autoimmune diseases, or thrombophilia risk factors. The risk of recurrent PIH in subsequent pregnancies is approximately thirty-three percent (33%), and PIH is superimposed in twenty-five percent (25%) of pregnancies in which chronic hypertension is present before pregnancy.

It is believed PIH occurs in about five to ten percent of all human pregnancies. PIH disorders are a leading global cause of maternal and infant illness and death. PIH occurs in over six million births a year and is responsible for 15 percent of all premature births. By conservative estimates, these disorders are responsible for 76,000 deaths each year. The risk of death for a pregnant woman with severe preeclampsia is 0.5%, and the risk of perinatal death for her baby is 13%; if the condition remains untreated and eclampsia develops, the risk of maternal and fetal death increases to 5% and 28%, respectively (see, Zupan J., Perinatal Mortality in Developing Countries, New Engl J. Med., 352(20) 2047-248 (2005), herein incorporated by reference in its entirety).

PIH is a syndrome having maternal and fetal manifestations. The maternal condition is characterized by vasospasm, activation of the coagulation system, oxidative stress and inflammatory-like responses, all of which have detrimental effects on the placenta, kidney, blood, liver, vasculature, cardiopulmonary system and brain. Intrauterine growth restriction or retardation ("IUGR") and intrauterine fetal demise are fetal symptoms of or complications associated with PIH. IUGR is the second leading cause of perinatal morbidity and mortality, and it occurs in approximately 5% of the general obstetric population. Placental insufficiency, preeclampsia, and abnormal placentation are generally recognized by those skilled in the art as being among the causes of or contributory factors in IUGR.

Women who develop mild gestational hypertension after 37 weeks gestation have pregnancy outcomes similar to those of pregnant women who are normotensive, apart from increased rates of induced labor and cesarean delivery. Conversely, women with severe gestational hypertension have high rates of placental abruption, preterm delivery and small-for-gestational-age babies (the postnatal counterpart and likely result of IUGR) similar to those of women with severe preeclampsia. Twenty-five percent (25%) of cases of eclampsia occur postpartum, usually in the first 2 to 4 days after delivery.

After a diagnosis of severe PIH, the baby is generally induced and delivered if it is near term, i.e., after 36 weeks. However, if PIH occurs earlier in the pregnancy, its impact is more profound because fetal viability is low; infant death occurs in approximately 87% of these cases. For pregnancies in which PIH occurs earlier than 24 weeks, the induction of labor is recommended and results in essentially 100% neonatal mortality. For pregnancies between 24 and 28 weeks gestation, management of PIH may be attempted to increase gestational age, provided that there is close monitoring for maternal and fetal complications. Regardless of gestational age of the fetus, delivery is the management method of choice for eclampsia.

There is no cure or effective treatment for PIH or IUGR. Delivery of the baby and placenta usually resolves the maternal symptoms of PIH within twelve (12) weeks postpartum. However, if the baby is not near term then early delivery is generally contrary to the best interests of the baby. Prophylactic measures against PIH, including calcium supplementation, vitamin and antioxidant supplementation and aspirin therapy, have not proven to be successful. Depending upon the stage of the pregnancy and the severity of maternal and fetal conditions, gestational hypertension, preeclampsia, eclampsia, and IUGR may be managed in an attempt to prolong the pregnancy and advance the gestational age to improve the fetal outcome. If maternal symptoms persist after delivery, management of PIH symptoms is necessary to prevent deterioration of the maternal condition or further development of complications.

Traditional management of PIH includes bed rest and antihypertensive and/or anticonvulsant therapy, including, without limitation, hydralazine, nifedipine, sodium nitroprusside, 1-methyldopa (e.g., ALDOMET), atenolol, labetalol, magnesium sulfate and phenyloin. Management of IUGR includes treatment of the concomitant maternal disease (e.g., gestational hypertension or preeclampsia) and bed rest. Preterm delivery may be necessary to prevent intrauterine demise due to chronic fetal oxygen deprivation or if the maternal condition does not respond to management efforts. Such therapies and procedures may be administered or prescribed based upon finding elevated (e.g., above 99^{th} percentile for normal patients) or cardiac Troponin I using the methods and systems described herein.

### II. Biological Samples

Samples or biological samples as used interchangeably herein include, but are not necessarily limited to, bodily fluids such as blood-related samples (e.g., whole blood, serum, plasma, and other blood-derived samples), urine, cerebral spinal fluid, bronchoalveolar lavage, and the like. Another example of a biological sample is a tissue sample. A biological sample may be fresh or stored (e.g. blood or blood fraction stored in a blood bank). The biological sample may be a bodily fluid expressly obtained for the assays of this invention or a bodily fluid obtained for another purpose which can be sub-sampled for the assays of this invention. In certain embodiments, the biological sample is whole blood. Whole blood may be obtained from the subject using standard clinical procedures. In other embodiments, the biological sample is plasma. Plasma may be obtained from whole blood samples by centrifugation of anti-coagulated blood. Such process provides a buffy coat of white cell components and a supernatant of the plasma. In certain embodiments, the biological sample is serum. Serum may be obtained by centrifugation of whole blood samples that have been collected in tubes that are free of anti-coagulant. The blood is permitted to clot prior to centrifugation. The yellowish-reddish fluid that is obtained by centrifugation is the serum. In another embodiment, the sample is urine. The sample may be pretreated as necessary by dilution in an appropriate buffer solution, heparinized, concentrated if desired, or fractionated by any number of methods including but not limited to ultracentrifugation, fractionation by fast performance liquid chromatography (FPLC), or precipitation of apolipoprotein B containing proteins with dextran sulfate or other methods. Any of a number of standard aqueous buffer solutions at physiological pH, such as phosphate, Tris, or the like, can be used.

### III. Subjects

The subject can be any pregnant human or other pregnant animal to be tested for characterizing its risk of CVD (e.g., eclampsia, pre-eclampsia, or gestational hypertension). In certain embodiments, the pregnant subject does not otherwise have an elevated risk of an adverse cardiovascular event (besides being pregnant), or has previously been diagnosed with CVD. Pregnant subjects having an elevated risk of experiencing a cardiovascular event include those with a family history of cardiovascular disease, elevated lipids, smokers, prior acute cardiovascular event, etc. (See, e.g., Harrison's Principles of Experimental Medicine, 15th Edition, McGraw-Hill, Inc., N.Y.--hereinafter "Harrison's").

In certain embodiments the subject is apparently healthy. The term "apparently healthy" as used herein, describes a subject who does not have any signs or symptoms of CVD, or has not previously been diagnosed as having any signs or symptoms indicating the presence of hypertension or atherosclerosis, such as angina pectoris, history of a cardiovascular event such as a myocardial infarction or stroke, or evidence of atherosclerosis by diagnostic imaging methods including, but not limited to, coronary angiography. Apparently healthy subjects also do not have any signs or symptoms of having heart failure or an aortic disorder.

In other embodiments, the pregnant subject already exhibits symptoms of cardiovascular disease. For example, the subject may exhibit symptoms of heart failure or an aortic disorder such as aortic dissection or aortic aneurysm. For subjects already experiencing cardiovascular disease, the levels of cTnI (e.g., compared to the 99^{th} percentile of a pregnant non-diseased control population), can be used to predict the likelihood of further cardiovascular events or the outcome of ongoing cardiovascular disease.

### IV. Exemplary Detection Assays

The present invention is not limited by the type of assay used to detect cardiac troponin I (cTnI). In certain embodiments, the methods for detecting troponin I are as described in U.S. Patent Application Publication No. 2012/0076803 and U.S. Patent No. 8,535,895.

In certain embodiments, an immunoassay is employed for detecting cTnI. Any suitable assay known in the art can be used, including commercially available cTnI assays. Examples of such assays include, but are not limited to, immunoassay, such as sandwich immunoassay (e.g., monoclonal-polyclonal sandwich immunoassays, including radioisotope detection (radioimmunoassay (RIA)) and enzyme detection (enzyme immunoassay (EIA) or enzyme-linked immunosorbent assay (ELISA) (e.g., Quantikine ELISA assays, R&D Systems, Minneapolis, Minn.)), competitive inhibition immunoassay (e.g., forward and reverse), fluorescence polarization immunoassay (FPIA), enzyme multiplied immunoassay technique (EMIT), bioluminescence resonance energy transfer (BRET), and homogeneous chemiluminescent assay, etc. In certain embodiments, cTnI is detected with the ERENNA detection assay system from Singulex Inc. or Abbott's hs TnI STAT^{®} ARCHITECT^{®} assay.

### EXAMPLES

The following examples are for purposes of illustration only and are not intended to limit the scope of the claims.

### EXAMPLE 1

### VARIATION OF HIGH-SENSITIVITY TROPONIN I LEVELS IN NORMAL PREGNANCY AND IN PREGNANCY COMPLICATED BY HYPERTENSION

This example describes examination of the variation in circulating concentrations of high-sensitivity troponin I (hs TnI) in the various trimesters of normal pregnancy and in pregnancy complicated by hypertension. Prospective data collection was carried out in the Department of Obstetrics and Gynaecology, Sultanah Aminah Hospital, Johor Bahru, Johor, Malaysia. All patients who fulfilled the inclusion criteria had their venous blood drawn and analyzed for cardiac troponin I using a high-sensitivity assay. These patients were grouped into different trimesters and postnatal. A total of 880 women were recruited over the study period. There were 129 patients in the first trimester, 207 in the second trimester, 416 in the third trimester, 90 in the postnatal period in normal uncomplicated pregnancy, 28 patients with gestational hypertension, and 10 patients with pre-eclampsia. Using very simple SPGS methods, the 99th percentiles of hs TnI were determined to be 4.0 pg/mL in the first trimester, 4.0 pg/mL in the second trimester, 6.0 pg/mL in the third trimester, and 28.0 pg/mL in the postnatal period. Using these same very simple SPGS methods, for gestational hypertension and preeclampsia, the 99th percentiles of hs TnI were 87 pg/mL and 783 pg/mL, respectively. Using more rigorous parametric methods, the 99th percentiles of hs TnI were determined to be 7.5 pg/mL in the first trimester, 4.6 pg/mL in the second trimester, 6.0 pg/mL in the third trimester, and 19.0 pg/mL in the postnatal period. Using these same parametric methods, for gestational hypertension and preeclampsia, the 99th percentiles of hs TnI were 58 pg/mL and 107 pg/mL, respectively. The observed values for overall normal pregnancy and normal gestational were much lower than the 99^{th} percentile for normal non-pregnant gender-specific females (15.6 pg/mL), whereas the gestational hypertension and pre-eclampsia groups were above the value.

### Methods

Study Population - All pregnant women who fulfilled the inclusion and exclusion criteria, presented to the Antenatal clinic, ward and labor room admission in Hospital Sultanah Aminah Johor Bahru, were included in the study. The inclusion criteria included pregnant women aged 18 to 35 years old, gravida 1 to 5 with uncomplicated antenatal history who gave verbal consent for the study. The exclusion criteria included: history of pre-existing hypertension, diabetes mellitus, haematological disorder, anaemia, blood transfusion, underlying cardiac disease (including congenital, acquired, cardiomyopathy or arrhythmia), documented ischaemic heart disease, previous cardiac surgery, and family history of cardiac disease or refusing consent.

Study Process - Patient information sheet was provided to the participant and verbal consent was taken to participate in the study. Study samples were then subdivided into each trimester accordingly for analysis. Venous blood samples were collected at the time of the clinic visit, admission to ward or immediate postnatal period. The blood was then sent to a laboratory for high-sensitivity cardiac troponin I analysis. The Abbott STAT ARCHITECT hs TnI assay was used (Abbott Diagnostics, Abbott Park, Illinois). Data were collected for demographic characteristics, current and past obstetric history, and intra-partum details. The outcome of interest was high-sensitivity troponin I level in pg/mL. Data were then analyzed using SPSS 21.0 for Windows (SPSS Inc., 2011). A p-value <0.05 was considered statistically significant. T-tests and analysis of variance (ANOVA) were used to test differences in the distribution of continuous variables. For the categorical variables, the differences were tested with chi-square test. For non-normally distributed continuous variables, Mann-Whitey U test was used for analysis. A multivariate logistic regression model was used to determine the association between variables identified and the high-sensitivity troponin I level for different subgroups. Colinearity between variables was assessed before including in the final model.

### Results

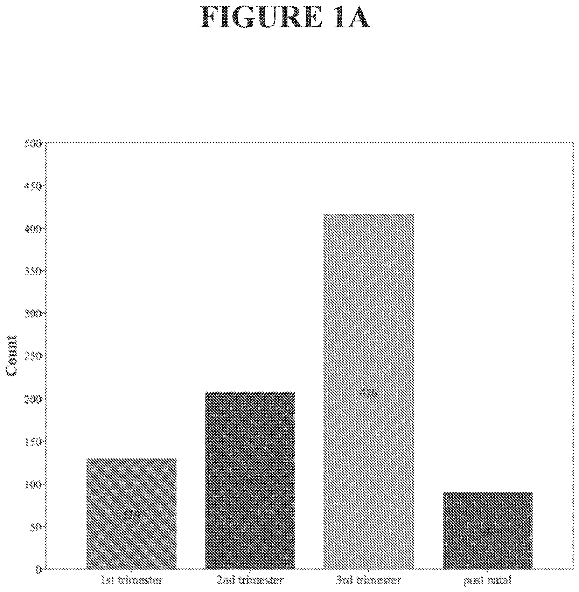

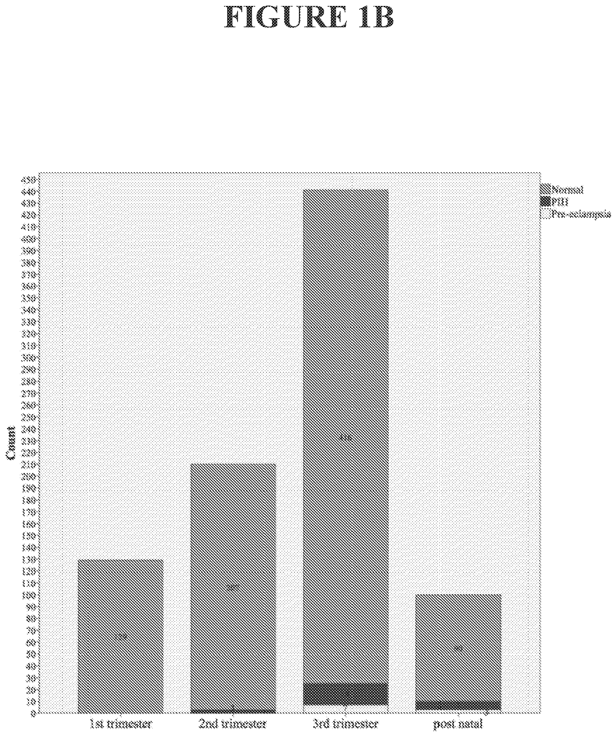

A total of 880 samples were obtained, with 842 of them from normal uncomplicated pregnancy, 28 from cases of gestational hypertension, and 10 from cases of preeclampsia. The 842 samples were divided into antenatal or immediate postnatal categories. Figure 1A shows total number of subjects recruited in the study in different trimesters and post-natal. Figure 1B shows the total number of normal healthy subjects with those with complicated pregnancy.

All four categories showed no significant differences in their demographic characteristics, as shown in Table 1.

**Table 1. Demographic Characteristics of Study Groups**

| | 1 st Trimester | 2nd Trimester | 3rd Trimester | Immediate postnatal | p value |
|---|---|---|---|---|---|
| | (n = 129) | (n = 207) | (n = 416) | (n = 90) | |
| Age* | 28.84 (5.29) | 28.96 (5.26) | 29.16 (4.98) | 29.76 (5.26) | 0.58 |
| Height (cm)* | 156.08 (5.71) | 156.10 (5.79) | 155.53 (5.97) | 156.45 (5.29) | 0.44 |
| Weight (kg)* | 61.70 (14.37) | 63.68 (14.57) | 64.48 (15.02) | 62.08 (13.02) | 0.23 |
| Ethnic^{#} | | | | | 0.07 |
| Malay | 94 (72.9) | 128 (61.8) | 259 (62.3) | 54 (60.0) | |
| Chinese | 10 (7.8) | 45 (21.7) | 86 (20.7) | 14 (15.6) | |
| Indian | 12 (9.3) | 15 (7.2) | 36 (8.7) | 10 (11.1) | |
| Others | 13 (10.1) | 19 (9.2) | 35 (8.4) | 12 (13.3) | |
| Conception^{#} | | | | | 0.55 |
| Spontaneous | 129 (100.0) | 205 (99.0) | 414 (99.5) | 90 (100.0) | |
| Assisted | 0(0) | 2 (1.0) | 2 (0.5) | 0(0) | |

| | | | | | |
|---|---|---|---|---|---|
| * Data presented as mean (SD) # Data presented as number (%) | | | | | |

The mean age group of all the pregnant subjects throughout pregnancy was below 30 years of age. The major ethnic group was Malays in all groups, 64.3%; followed by Chinese, 16.45%; Indian, 9.08% and others 10.17%. Almost all pregnancies were delivered spontaneously.

All three different gestational trimesters groups exhibited similar high sensitive Troponin I level (hsTnI), pg/mL with a p-value of 0.206, as shown in Table 2.

**Table 2. Troponin I level (pg/mL) according to different category**

| | mean | SD | 99^{th} percentile |
|---|---|---|---|
| Antenatal | | | |
| 1st trimester | 0.59 | 1.08 | 4.0 |
| 2nd trimester | 0.59 | 0.97 | 4.0 |
| 3rd trimester | 0.99 | 1.16 | 6.0 |
| Immediate Postnatal | 3.22 | 4.36 | 28.0 |

There was increasing trend of hsTnI in the immediate postnatal period which showed a significant difference as compared to the antenatal period, with a p-value < 0.001.

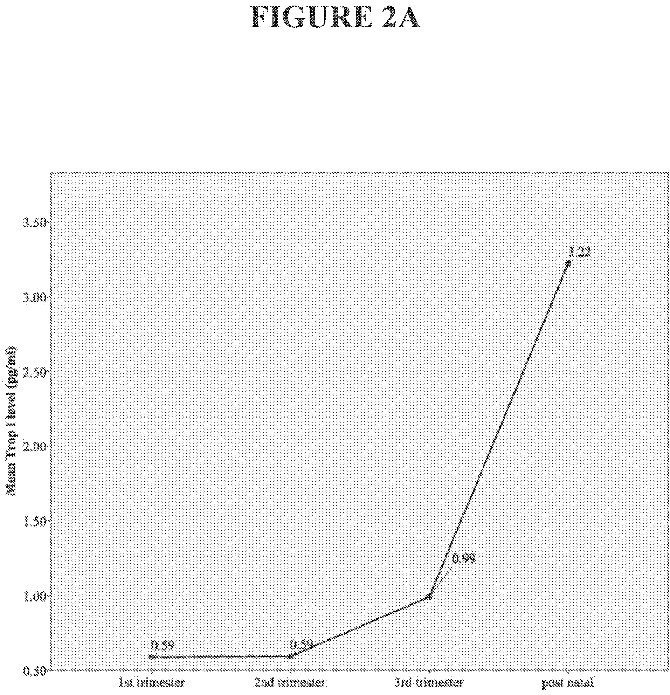

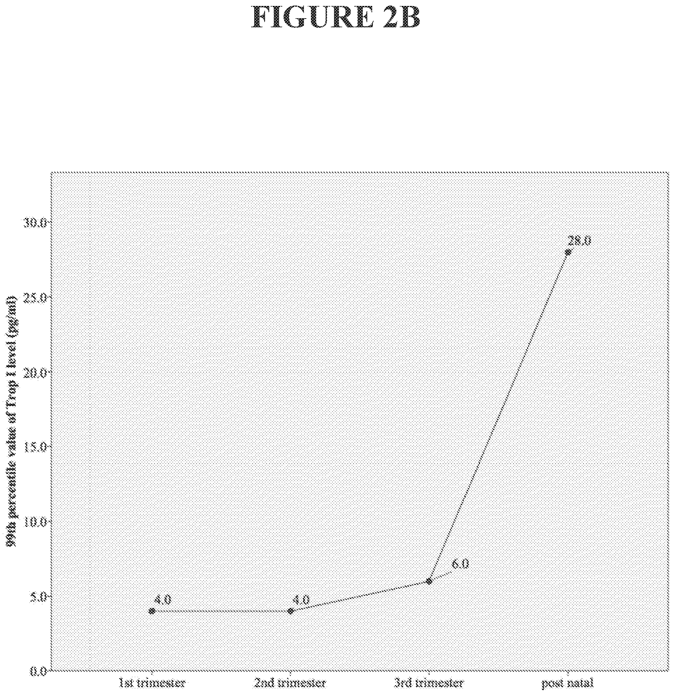

Figure 2A shows the mean value of Troponin I of uncomplicated pregnancy in different gestational categories. Figure 2B shows the 99^{th} percentile value of Troponin I of the uncomplicated pregnancy group in different gestational categories. The high-sensitivity Troponin I level showed a significant increasing trend in the study group with gestational hypertension and pre-eclampsia, as shown in Table 3

**Table 3. Troponin I level (pg/mL) in hypertensive complicated pregnancy**

| | mean | SD | 99^{th} percentile | p value* |
|---|---|---|---|---|
| Gestational Hypertension | 17.32 | 18.20 | 87 | 0.01 1 |
| Pre-eclampsia | 111.49 | 24.09 | 783 | <0.001 |

| | | | | |
|---|---|---|---|---|
| *** compare with normal uncomplicated pregnancy category** | | | | |

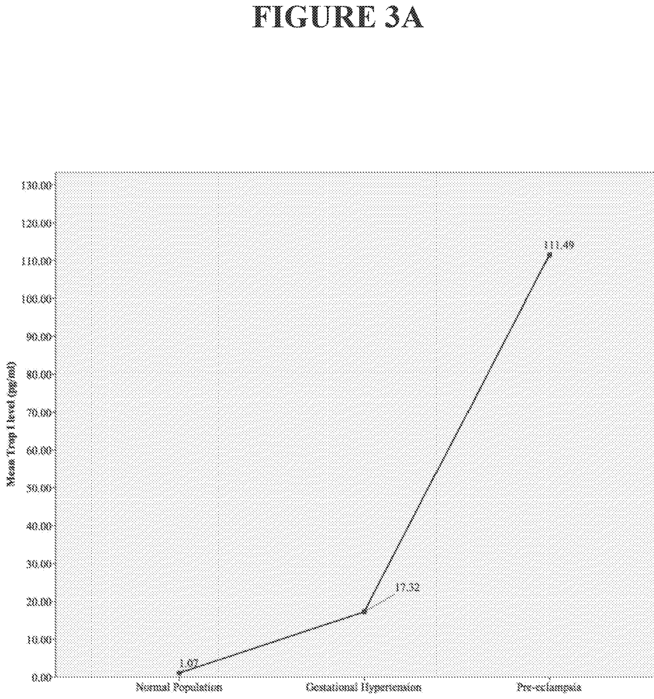

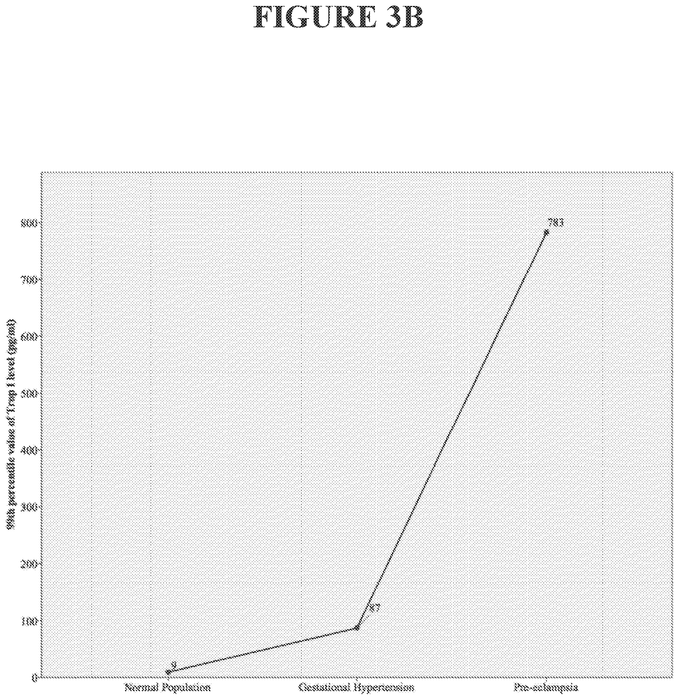

Figure 3A shows the mean value of troponin I compared with the normal group. Figure 3B shows the 99^{th} percentile value of troponin I compared with the normal group.

This example demonstrates the circulating levels of highly sensitive cardiac troponin I levels of patients with normotensive pregnancy and hypertension complicating pregnancy. The results of this example show that there was no significant difference in the hs TnI levels in all three trimesters, and the levels were below the gender-specific 99th percentile cut-off values.

It also was found that hs TnI was elevated immediately after postpartum, which is in contrast to the findings disclosed in Shivvers et. al., Am J Obstet Gynecol, 180:122, (1999) (15), i.e., that troponin I levels remain undetectable during and after delivery. The cause of the increment and its clinical significance for the pregnant woman are unknown and it was not possible to rule out the possibility of insulation toward heart during the labor process. Other possible influential factors including maternal age, BMI, ethnicity, parity, and mode of delivery showed no correlation toward hs TnI levels. In conclusion, serum cardiac hs TnI level remains low throughout the three trimesters, but were elevated during the immediate post-partum period. These findings are important in view of the major contribution in pregnancy to mortality and morbidity including hypertensive disorders in pregnancy.

### REFERENCES:

1. Kim E.B, Susan M.B, Scott B, Heddwen B, 2012, Ganong's Review of Medical Physiology, 24th Edition, McGraw-Hill Medical.
2. John E. Hall, Guyton and Hall; Textbook of Medical Physioogy, 2010, 12th Edition, Saunders
3. Kaur et al., Webmed Central PUBLIC HEALTH 201; 2(12): WMC002599.
4. Duvekot et al., Obstet Gynecol Surv 1994; 49: S1-14.
5. Lang et al., Am Heart J 1991; 121: 1768-1775.
6. Barton et al., Am J Obstet Gynecol 1991; 165: 389-391.
7. Kuzniar et al., Am J Obstet Gynecol 1983; 146: 400-405.
8. Perri SV, Biochem Soc Trans 1979; 7: 593-617.
9. Triquier et al. Clin Chem 1995; 41: 1710-1715.
10. Apple et al., Clin Chem 1997; 43: 2047-2051.
11. Missov et al., Coron Artery Dis 1997; 8: 537-541.
12. Redman CW & Sargent IL. Placenta 2009; 30(Suppl A): S38-S42.
13. Redman CW & Sargent IL. Placenta 2000; 21: 597-602.
14. Brown MA. Clin Exp Pharmacol Physiol 1995; 22:781-791.
15. Shivvers et al., Am J Obstet Gynecol 180:122, 1999.
16. Joyal et al., Am J Medicine (2007) 120, 819.e13-819. e14.
17. Fleming, et al., (2000). BJOG: An International Journal of Obstetrics & Gynaecology 107(11): 1417-1420.
18. Aboutaleb et al., Life Science Journal 2013;10(11s)
19. Shah, et al. (2015). BMJ (Clinical Research Ed.), 350, g7873.
20. Apple et al., Clinical Chemistry November 2012 vol. 58 no. 11 1574-1581

Although only a few exemplary embodiments have been described in detail, those skilled in the art will readily appreciate that many modifications are possible in the exemplary embodiments without materially departing from the novel teachings and advantages of this disclosure. Accordingly, all such modifications and alternative are intended to be included within the scope of the invention as defined in the following claims.

## Claims

1. A system comprising:
a) a report that:
i) provides a sample concentration of cTnI from a first, second, or third trimester for a pregnant woman as tested by an immunoassay ;
ii) indicates that the sample concentration of cTnI from said first, second, or third trimester is higher than a 99th percentile cTnI concentration from a corresponding first, second, or third trimester uncomplicated pregnancy control group as tested by the immunoassay, wherein the 99th percentile cTnI concentration for said first trimester is about 4.0 pg/ml, the 99th percentile cTnI concentration for said second trimester is about 4.0 pg/ml, and the 99th percentile cTnI concentration for said third trimester is about 6.0 pg/ml: and
iii) indicates that said pregnant woman has, or is at risk of developing, cardiovascular disease (CVD) or a complication of CVD; and
b) a computer for generating said report, which computer comprises:
i) a computer processor for receiving, processing, and communicating data,
ii) a storage component for storing data which contains a reference database containing said 99th percentile cTnI concentration for said first, second, and third trimester uncomplicated pregnancy control groups; and
iii) a computer program embedded within said computer processor, which is configured to process said sample concentration of cTnI in the context of said reference database to determine, as an outcome, if said pregnant woman has, or is at risk for developing, CVD or a complication of CVD.

2. The system of claim 1, wherein said report provides the 99th percentile cTnI concentration for all three of said first, said second, and said third trimester uncomplicated pregnancy control group as tested by the immunoassay.

3. The system of claim 1 or claim 2, further comprising a cardiovascular disease (CVD) therapeutic.

## Patentansprüche

1. Ein System, das Folgendes umfasst:
a) einen Bericht, der:
i) eine Probenkonzentration von cTnI aus einem ersten, zweiten oder dritten Trimester für eine schwangere Frau, getestet durch einen Immunassay, angibt;
ii) angibt, dass die Probenkonzentration von cTnI aus dem ersten, zweiten oder dritten Trimester höher ist als eine 99. Perzentile-cTnI-Konzentration aus dem entsprechenden ersten, zweiten oder dritten Trimester einer Kontrollgruppe mit unkomplizierter Schwangerschaft, getestet durch den Immunassay, wobei die 99. Perzentile-cTnI-Konzentration für das erste Trimester ungefähr 4,0 pg/ml beträgt, die 99. Perzentile-cTnI-Konzentration für das zweite Trimester ungefähr 4,0 pg/ml beträgt und die 99. Perzentile-cTnI-Konzentration für das dritte Trimester ungefähr 6,0 pg/ml beträgt; und
iii) angibt, dass die schwangere Frau eine Herz-Kreislauf-Erkrankung, cardiovascular disease - CVD -, oder eine Komplikation von CVD hat oder bei ihr das Risiko besteht, eine solche zu entwickeln; und b) einen Computer zur Erstellung des Berichts, wobei der Computer Folgendes umfasst:
i) einen Computerprozessor zum Empfangen, Verarbeiten und Übertragen von Daten,
ii) eine Speicherkomponente zum Speichern von Daten, die eine Referenzdatenbank enthält, welche die 99. Perzentile-cTnI-Konzentration für das erste, zweite und dritte Trimester der Kontrollgruppen mit unkomplizierter Schwangerschaft enthält; und
iii) ein Computerprogramm, eingebettet in den Computerprozessor, das konfiguriert ist, um die Probenkonzentration von cTnI im Kontext der Referenzdatenbank zu verarbeiten, um als Ergebnis zu bestimmen, ob die schwangere Frau CVD oder eine Komplikation von CVD hat oder ob bei ihr das Risiko besteht, eine solche zu entwickeln.

2. Das System gemäß Anspruch 1, wobei der Bericht die 99. Perzentile-cTnI-Konzentration sowohl für das erste als auch für das zweite und das dritte Trimester der Kontrollgruppe mit unkomplizierter Schwangerschaft, getestet durch den Immunassay, angibt.

3. Das System gemäß Anspruch 1 oder Anspruch 2, das weiter ein Therapeutikum für Herz-Kreislauf-Erkrankung, CVD, umfasst.

## Revendications

1. Système comprenant:
a) un rapport qui:
i) fournit une concentration de cTnI dans un échantillon d'un premier, deuxième ou troisième trimestre pour une femme enceinte comme testé par un test immunologique;
ii) indique que la concentration de cTnI dans l'échantillon dudit premier, deuxième ou troisième trimestre est supérieure à une concentration de cTnI au 99^{ème} percentile d'un groupe témoin de grossesse non compliquée au premier, deuxième ou troisième trimestre correspondant, comme testé par le test immunologique, dans lequel la concentration de cTnI au 99^{ème} percentile pour ledit premier trimestre est d'environ 4,0 pg/ml, la concentration de cTnI au 99^{ème} percentile pour ledit deuxième trimestre est d'environ 4,0 pg/ml et la concentration de cTnI au 99^{ème} percentile pour ledit troisième trimestre est d'environ 6,0 pg/ ml; et
iii) indique que ladite femme enceinte a, ou risque de développer, une maladie cardiovasculaire (MCV) ou une complication de MCV; et
b) un ordinateur pour générer ledit rapport, lequel ordinateur comprend:
i) un processeur informatique pour recevoir, traiter et communiquer des données,
ii) un composant de stockage pour stocker des données qui contient une base de données de référence contenant ladite concentration de cTnI au 99^{ème} percentile pour lesdits groupes témoins de grossesse non compliquée au premier, deuxième et troisième trimestre; et
iii) un programme informatique intégré dans ledit processeur informatique, qui est configuré pour traiter ladite concentration de cTnI dans l'échantillon dans le contexte de ladite base de données de référence pour déterminer, comme résultat, si ladite femme enceinte a, ou risque de développer, une MCV ou une complication de MCV.

2. Système selon la revendication 1, dans lequel ledit rapport fournit la concentration de cTnI au 99^{ème} percentile pour les trois dudit groupe témoin de grossesse non compliquée au premier, deuxième et troisième trimestre, comme testé par le test immunologique.

3. Système selon la revendication 1 ou la revendication 2, comprenant en outre une thérapeutique des maladies cardiovasculaires (MCV).
